Europäisches Patentamt

European Patent Office

Office européen des brevets

(11) Veröffentlichungsnummer: **0 321 596 B1**

(12) **EUROPÄISCHE PATENTSCHRIFT**

(45) Veröffentlichungstag der Patentschrift: **15.04.92**

(51) Int. Cl.⁵: **C05F 9/02**

(21) Anmeldenummer: **87118997.3**

(22) Anmeldetag: **22.12.87**

(54) Vorrichtung zum Herstellen von Humus.

(43) Veröffentlichungstag der Anmeldung:
**28.06.89 Patentblatt 89/26**

(45) Bekanntmachung des Hinweises auf die
Patenterteilung:
**15.04.92 Patentblatt 92/16**

(84) Benannte Vertragsstaaten:
**AT BE CH DE FR IT LI NL**

(56) Entgegenhaltungen:
**DE-A- 2 460 494        DE-A- 3 431 049**
**DE-U- 8 530 923        FR-A- 2 594 434**
**GB-A- 1 321 416        LU-A- 85 188**
**US-A- 3 961 603**

(73) Patentinhaber: **Reuss, Peter**
**Schlesienstrasse 10**
**W-8602 Memmelsdorf(DE)**

Patentinhaber: **Reuss, Rita**
**Schlesienstrasse 10**
**W-8602 Memmelsdorf(DE)**

(72) Erfinder: **Reuss, Peter**
**Schlesienstrasse 10**
**W-8602 Memmelsdorf(DE)**
Erfinder: **Reuss, Rita**
**Schlesienstrasse 10**
**W-8602 Memmelsdorf(DE)**

(74) Vertreter: **Göbel, Matthias, Dipl.-Ing.**
**Pruppacher Hauptstrasse 5-7**
**W-8501 Pyrbaum-Pruppach(DE)**

## Beschreibung

Die Erfindung betrifft eine Vorrichtung zum Herstellen von Humus mit einem einen Bodenteil mit Zuluftöffnung, einem aus Elementen schachtartig aufgebauten Mittelteil sowie einem Deckelteil mit einer Einfüllöffnung gebildeten Aufnahmebehälter für die Ausgangsmaterialien.

Bei einer bekannten Kompostierungszelle mit den zuvor genannten Merkmalen (DE-U-8530923) ist einer im Bodenteil angeordneten Entleerungsöffnung eine manuelle Austragsraspel oder eine Fräswalze zugeordnet, wodurch zum Zwecke einer ausreichenden Belüftung der im Querschnitt eingeschränkten Entleerungsöffnung ein Gebläse erforderlich ist.

Es ist Aufgabe der Erfindung bei einer Vorrichtung obiger Gattung eine einfache regelbare Luftzuführung in den Aufnahmebehälter sowie Maßnahmen zur Bereitstellung eines Wurmbesatzes zu schaffen.

Der Erfindung gemäß ist diese Aufgabe dadurch gelöst, daß der Bodenteil mindestens eine durch eine gelochte Platte fest übergriffene Aussparung und eine durch eine verschiebliche ungelochte Platte übergriffe Aussparung aufweist, daß in den Aussparungen Schubladen verschieblich eingestellt sind, daß die der gelochten Platte zugeordnete Schublade im Abstand hintereinander eine äussere sich über die volle Höhe der Aussparung erstreckende Querwand und eine innere über eine Teilhöhe sich erstreckende Querwand aufweist und daß der Boden dieser Schublade zwischen den beiden Querwänden offen ausgebildet ist. Zweckmäßig weist der Bodenteil eine durch eine ungelochte Platte übergriffene Aussparung und beidseitig zu dieser im Abstand je eine durch gelochte Platten übergriffene Aussparung auf. Vermittels der Schubladen sind durch teilweises Herausziehen derselben Zuluftkanäle mit regelbarem Einfluß auf die Luftzuführung erzielt, während die der ungelochten Platte zugeordnete Schublade die Bereitstellung eines Wurmbesatzes ermöglicht. Durch Verschieben der ungelochten Platte in der Plattenebene der Aussparung bzw. der Schublade können Regenwürmer oder Regenwürmerbrut in den Innenraum des Aufnahmebehälters wandern. Der Benutzer kann so bei Erreichen einer für die Regenwürmer verträglichen Rottewärme im Aufnahmebehälter durch eine einfache Ziehbewegung den Weg für den Wurmbesatz des Aufnahmebehälters freigeben.

Zweckmäßig ist der Bodenteil, die aufbaubaren Elemente und der Deckelteil aus einem Isolierwerkstoff gebildet. Als geeigneter Isolierwerkstoff kann ein ge-schäumter Kunststoff oder ein Fasernwerkstoff dienen. Durch die Anordnung einer mehr oder weniger großen An-zahl von Elementen ist eine im Fassungsvermögen beliebig veränderliche Vorrichtung geschaffen, die den Humus und das Ausgangsmaterial gegen schädigende Austrocknung bzw. Verwässerung, Hitze oder Kälte schützt, wodurch permanent über die Jahreszeiten hinweg eine Humusherstellung möglich ist. Außerdem sichert der Aufnahmebehälter den Wurmbesatz gegen Vernichtung durch Witterungseinflüsse, wie Kälte und Hitze und verhindert ein Abwandern des Wurmbesatzes ins Erdreich.

Es versteht sich, daß der durch den Bodenteil, die Elemente und den Deckelteil gebildete Aufnahmebehälter auch aus einem beliebig anderweitig geeigneten Werkstoff, z.B. solchem mit großer Festigkeit, wie Metall, Holz oder Kunststoff gebildet sein kann. Vermittels des Dekkelteils wird darüberhinaus ein Schutz gegen Austrocknen des Behälterinhalts geschaffen und eine Feuchtigkeitsregelung für diesen erzielt.

In Ausgestaltung der Vorrichtung ist vorgesehen, daß der Bodenteil, die Elemente und der Deckelteil in den jeweils gemeinsamen Trennungsflächen korrespondierende Nuten und Federn aufweisen und daß die Nuten und Federn den Bodenteil, die Elemente und den Deckelteil quer zur aufrechten Längsachse des Aufnahmebehälters gegeneinander fixieren. Zweckmäßig sind die in den Trennungsflächen angeordneten Nuten und Federn umlaufend ausgebildet. Es versteht sich, daß auch andere Mittel, z.B. Verzahnungen für eine Ausrichtung und Fixierung von Bodenteil und Elementen bzw. Deckelteil in Anwendung gebracht werden können.

Ferner ist vorgesehen, in den gemeinsamen Trennungsflächen von Bodenteil, Elementen und Deckelteil abschnittsweise Abstandsstücke anzuordnen, die zwischen sich zusätzliche Zuluftöffnungen für die Außenluft bilden. Vorzugsweise sind die Abstandstücke in den Eckbereichen der gemeinsamen Trennungsflächen von Bodenteil, Elementen und Deckelteil angeordnet. Über die Zuluftöffnungen sind intensive partielle oder insgesamt Belüftungen des Behälterinhalts möglich und Regulierungen des Luftbedarfs durchführbar, was sich auf die Bekterientätigkeit und den Kleinlebewesen in den zuverrottenden Materialien günstig auswirkt.

Der Bodenteil und die Elemente tragen zweckmäßig innen über die gemeinsame Begrenzungsflächen benachbarter Bauteile ragende gelochte Streifen, die mittels in Aussparungen der Begrenzungsflächen einragenden Anformungen am Bodenteil oder den Elementen festlegbar sind. Die gelochten Streifen sind dabei so ausgebildet, daß bei abstandslosen Zuordnungen von Bodenteil, Elementen und Deckelteil diese an den Innenwandungen wirkungsfrei vorbei greifen, während bei Anwendung der Abstandsstücke die Zuluftöffnungen Humusteilchen bzw. Regenwürmer an einem Herausfallen aus dem Aufnahmebehälter sichern.

Weiterhin ist vorgesehen, den Deckelteil insbesondere mit einer durch eine Klappe oder einen Stülpdeckel verschließbaren Öffnung zu versehen. Der Klapp- oder Stülpdeckel erlaubt durch Öffnen die Abführung überschüssiger Wärme an die Außenluft und ein einfaches Nachfüllen von Ausgangsmaterialien, insbesondere bei größeren Aufnahmebehältern. Von besonderem Vorteil hat sich jedoch erwiesen, wenn der Deckel in der Öffnung einen mit einer Schneid- oder Schlageinrichtung versehenen Häcksler für die Ausgangsmaterialien aufweist. Die Ausgangsmaterialien erhalten durch die Zerkleinerung eine Aufbereitung, was sich auf die Bakterientätigkeit im Aufnahmebehälter günstig auswirkt. Außerdem ist noch vorgesehen, den Bodenteil an seiner Unterseite mit als Stützfüße dienenden Ansätzen, Anformungen od.dgl. zu versehen. Zwischen den Stützfüßen können Einhängeschlitze für Tragegriffe ausgebildet sein, die den Transport der Vorrichtung erleichtern helfen.

Um auch über kalte Jahreszeiten hinweg eine ausreichende Sicherung der im Aufnahmebehälter erforderlichen Wärme zu gewahrleisten, ist nach weiterer Erfindung vorgesehen, in den durch gelochte Platten übergriffenen Aussparungen oder den in diesen untergebrachten Schubladen Wärmeelemente austauschbar unterzubringen. Als geeignete Wärmeelemente können elektrische Heizkörper oder wärmespeichernde Elemente, beispielsweise solche aus Ton, Schamotte oder Keramik dienen.

Schließlich können zur Nutzung der im Aufnahmebehälter sich durch die Bakterientätigkeit einstellenden Verrottungswärme die Elemente Stecköffnungen für die Durchführung von, z.B. mit einer Wärmepumpe verbundenen Warmezellen aufweisen. Es ist denkbar, die Wärmezellen jeweils in die bei der Verrottung wärmsten Zonen einschließenden Elementen einzuschieben. Diese Stecköffnungen sind durch ein Verschlußglied, z.B. Stöpsel oder Klappe abdeckbar.

Als besonders vorteilhaft hat sich erwiesen, wenn der Bodenteil und/oder die Elemente sowie der Deckelteil innen mit einem Folienwerkstoff, z.B. einer Alufolie kaschiert sind. Die Folie kann als Warmefolie wirken und dadurch den Wärmehaushalt der Vorrichtung stabilisieren. Außerdem ist noch denkbar, den Bodenteil, die Elemente und den Deckelteil außen mit einer Wetterschutzauflage, z.B. einem Lackauftrag zu versehen.

Es versteht sich, daß die Vorrichtung nicht auf eine Größe festgelegt ist. Auch sind verschiedene Querschnittsformen für die Vorrichtung denkbar, z.B. sind eckige oder runde Querschnittsformen in Anwendung bringbar.

Die Erfindung ist anhand von Ausführungsbeispielen in der Zeichnung verdeutlicht. Es zeigen:

Fig. 1     eine Vorrichtung in Vorderansicht,

Fig. 2     eine Vorrichtung im Schnitt nach der Linie II-II der Fig. 1,

Fig. 3     eine Vorrichtung im Schnitt,

Fig. 4     eine Vorrichtung abgewandelter Ausführung in Vorderansicht,

Fig. 5     einen Bodenteil mit offenen Aussparungen in Draufsicht,

Fig. 6     einen Bodenteil mit abgedeckten Aussparungen in Draufsicht,

Fig. 7     ein Element in Draufsicht,

Fig. 8     ein Element anderer Ausführung in Draufsicht,

Fig. 9     ein Teilstück eines Elements der Fig. 7, vergrößert,

Fig. 10     ein Teilstück eines Elements der Fig. 8, vergrößert,

Fig. 11     einen Teilschnitt des Bereichs einer Trennungsfläche von zwei Elementen, vergrößert,

Fig. 12     ein Teilstück des Bereichs einer angehefteten Trennungsfläche von zwei Elementen, vergrößert,

Fig. 13     einen Teilschnitt von Elementen, Abstandsstücken und Lochstreifen, vergrößert,

Fig. 14     eine weitere Vorrichtung in Vorderansicht,

Fig. 15     eine Vorrichtung der Fig. 14, im Schnitt,

Fig. 16     einen weiteren Schnitt der Vorrichtung der Fig. 14 und

Fig. 17     eine Vorrichtung gemäß Fig. 14 in Draufsicht.

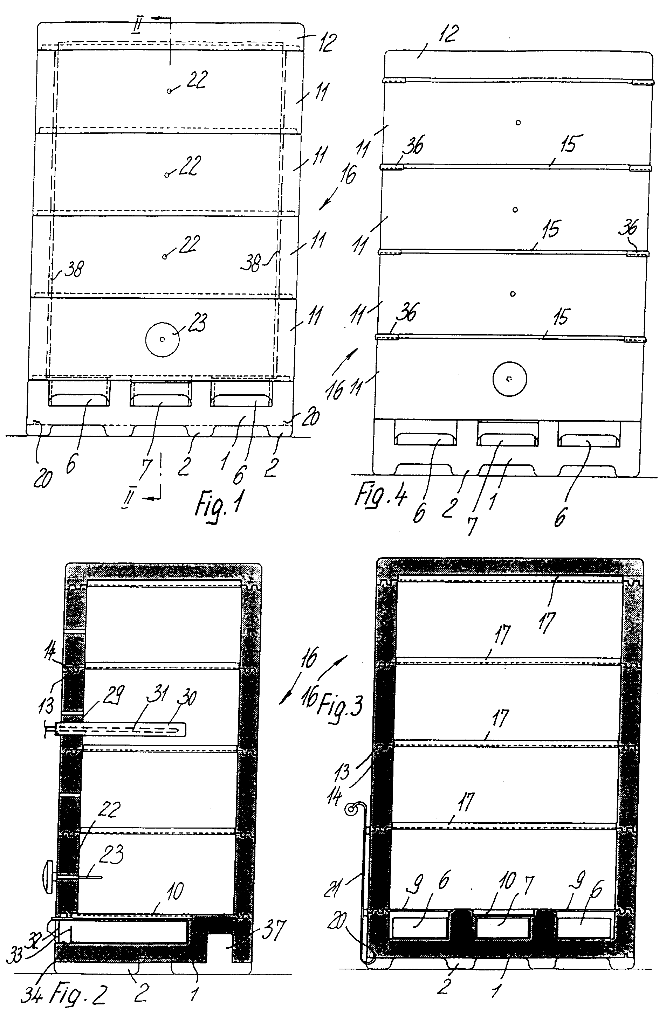

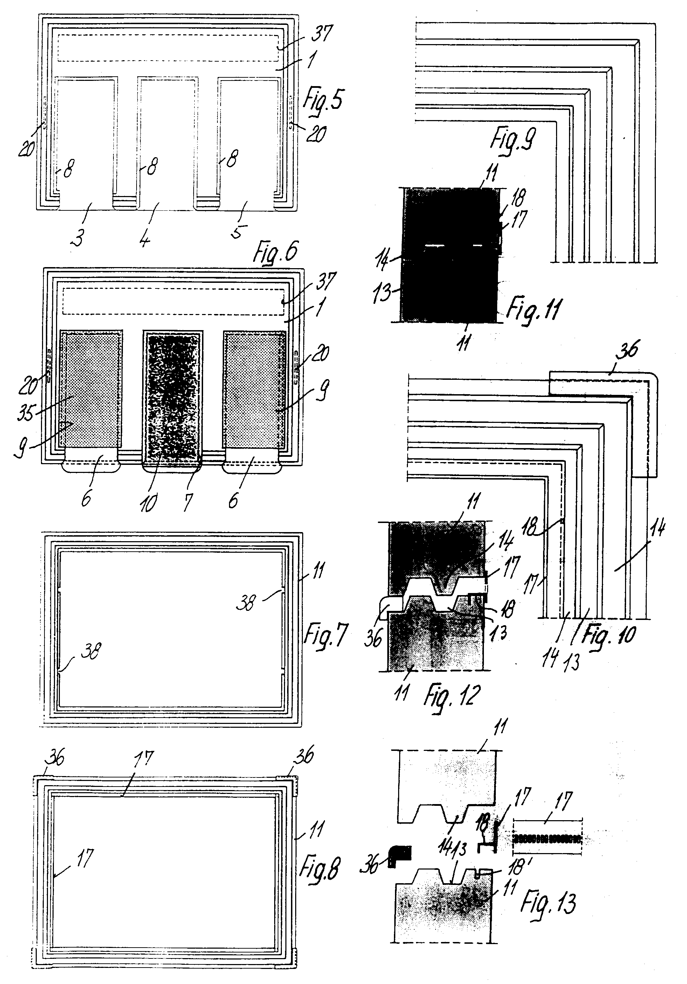

In den Fig. ist mit 1 ein im wesentlichen ebener Bodenteil bezeichnet, der mittels Fußstützen 2 auf Aufstellflächen aufstellbar ist und im Bereich der Oberseite nebeneinander prismatische Aussparungen 3, 4 und 5 (Fig. 5) aufweist. Die Aussparungen 3, 4 und 5 nehmen Schubladen 6 und 7 auf. In den Randflächen der Aussparungen 3 bis 5 sind Absetzungen 8 für die Stützung von je einer gelochten Platte 9 und in den Randflächen der Aussparung 4 in Absetzungen 8 eine ungelochte Platte 10 (Fig. 6) verschieblich angeordnet. Auf den Bodenteil 1 sind eine Anzahl Elemente 11 frei aufstellbar, die gemeinsam mit dem Bodenteil 1 einen Aufnahmebehälter 16 für Humus bzw. Ausgangsmaterial bilden. Das dem Bodenteil 1 abgewandte Element 11 trägt einen frei aufgelegten Deckelteil 12. Der Bodenteil 1, die Elemente 11 und der Deckelteil 12 sind bevorzugt aus einem geschäumten Kunststoff, z.B. Polystyrol gebildet. Zur seitlichen Fixierung der Elemente 11 zum Bodenteil 1 bzw. untereinander und gegenüber dem Deckelteil 12 sind in den jeweils gemeinsamen Begrenzungsflächen Nuten 13 (Fig. 2, 3 und 11, 12, 13) und Federn 14 umlaufend eingeformt bzw. eingeschnitten. Während beim Ausführungsbeispiel der Fig. 1 bis 3 die Elemente 11 sich unmittelbar (Fig. 11) aufeinander und dem Bodenteil 1 abstützen, sind die Elemente 11 des Ausführungsbeispiels der Fig. 4 durch in den Eckbereichen (Fig. 10, 12 und 13) angeordnete Abstandsstücke 36 im Abstand voneinander gehalten. Die so gebildeten Luftschlitze 15 erlauben eine partielle Belüftung des Innenraums zwischen den Elementen 11 bzw. zwischen diesen und dem Bodenteil 1 des Aufnahmebehälters 16. Zur Verhinderung des Herausfallens von Teilen des Inhalts des Aufnahmebehälters 16 sind die Luftschlitze 15 durch Lochstreifen 17 überdeckt, die durch Eingreifen von Hakenteilen 18 in Schlitzungen 19 der Elemente 11 bzw. des Bodenteils 1 fixiert sind.

Unterseitig weist der Bodenteil 1 Schlitzungen 20 auf, in die Tragegriffe 21 (Fig. 15) einhängbar sind, die das Anheben und den Transport der Vorrichtung erleichtern. Die Elemente 11 sind frontseitig mit Öffnungen 22 versehen, die das Einbringen von Temperaturmeßeinrichtungen 23 in den Innenraum des Aufnahmebehälters 16 erlauben. Mittels der Temperaturmeßeinrichtungen 23 kann der Benutzer in beliebiger Höhe des Aufnahmebehälters 16 die dort jeweils vorhandene Temperatur, die durch die Verrottungswärme bestimmt wird, erkennen.

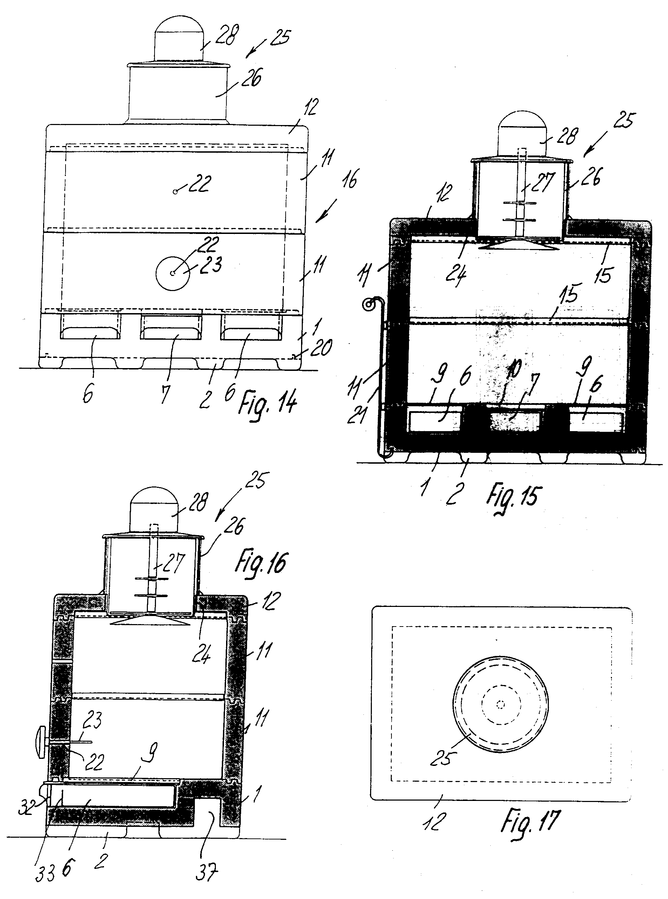

Während beim Ausführungsbeispiel der Fig. 1 bis 4 ein flach ausgebildeter geschlossener Deckelteil 12 in Anwendung gebracht ist, der zu Zwecken des Auffüllens des Aufnahmebehälters 16 abzunehmen ist, ist der Deckelteil 12 der Ausführungsform der Fig. 14 bis 17 mit einer Öffnung 24 versehen, die durch einen Häcksler 25 übergriffen ist. Der Häcksler 25 weist ein zylindrisches Gehäuse 26 auf, in dem eine Schneideinrichtung 27 drehbar ist. Beim Auffüllen der Vorrichtung gelangen die Ausgangsstoffe nach dem Abnehmen der Abdeckung 28 an die Schneideinrichtung 27 und werden durch die Schneideinrichtung 27 in eine für die Humusherstellung vorteilhafte Größe aufbereitet.

Schließlich können die Elemente 11 Öffnungen 29 für die steckbare Aufnahme von Wärmeelementen 30 aufweisen, die mit ihrem Rohrsystem 31 in den Innenraum des Aufnahmebehälters 16 einragen und an eine Wärmepumpe (nicht gezeigt) zur Nutzung der Verrottungswärme anliegen.

Zur Herstellung von Humus ist davon auszugehen, daß nach Abnahme des Deckelteils 12 oder über den Häcksler 25 der Aufnahmebehälter 16 mehr oder weniger mit Ausgangsmaterial, z.B. Küchenabfällen, Gräsern oder Laubteilen auffüllbar ist. Die Schubladen 6 und 7 befinden sich in ihrer inneren Endstellung und sind durch die Platten 9 und 10 übergriffen. Bei der einsetzenden Verrottung kann durch teilweises Herausziehen der Schubladen 6 eine Frischluftzufuhr an den Innenraum des Aufnahmebehälters 16 bewirkt werden. Die Schubladen 6 weisen hierzu, wie insbesondere aus der Fig. 2 erkennbar, zwei im Abstand hintereinander aufrecht stehende Querwände 32 und 33 auf, wobei die Querwand 33 mit geringerer Höhe ausgeführt ist. Durch die zusätzliche Anordnung eines zwischen den Querwänden 32, 33 offenen Bodenabschnitts 34 können bei einer Teilausziehbewegung der Schubladen 6 über die Bodenabschnitte 34 und zwischen den Querwänden 32, 33 Luftströmungen in die Schubladen 6 und von dort über die Lochungen 35 der Platten 9 in den Aufnahmebehälter 16 gelangen. ALs Lochplatten 9 kann z.B. ein Siebgewebe dienen. Soll eine zusätzliche Belüftung des Inhalts des Aufnahmebehälters 16 erfolgen, so ist dies durch Einbringen von Abstandsstücken 36 in die Eckbereiche zwischen den Elementen 11 und den so erzielten Luftschlitzen 15 möglich.

Nach dem Nachlassen der sich zu Beginn der Verrottung einstellenden hohen Verrottungstemperaturen kann durch Herausziehen der Platte 10 die mittlere Schublade 7 freigelegt und mit dem Innenraum des Vorratsbehälters 16 in Verbindung gebracht werden. In die Schublade 7 bevorratete Wurmbrut bzw. Regenwürmer können so in die mehr oder weniger verrotteten Ausgangsstoffe zur Unterstützung der Humusbildung eindringen und dort die Humusherstellung unterstützen bzw. sich vermehren. Die Verwendung von wärmeisolierenden Werkstoffen für den Bodenteil 1, den Elementen 11 und den Deckelteilen 12 erbringt die Beibehaltung einer für die Tätigkeit der Regenwürmer ausreichenden Wärme im Aufnahmebehälter 16, so daß auch ggf. unterstützt durch Wärmeelemente (nicht gezeigt) bei kalten Jahreszeiten die Erzeugung von Humus und die Zucht von Regenwürmern möglich ist.

Eine in Bodenteil 1 eingearbeitete Ausnehmung 37 vermeidet Materialanhäufungen und erleichtert das Ausformen des Bodenteils 1. Zur Bildung einer Luftströmung im Innenraum des Aufnahmebehälters 16 sind die Elemente 11 im Bereich der inneren Begrenzungsfläche mit Kanälen 38 versehen, die zum Innenraum hin, bevorzugt über ihre ganze Länge offen sind.

## Patentansprüche

1. Vorrichtung zum Herstellen von Humus mit einem durch einen Bodenteil mit Zuluftöffnung, einem aus Elementen aufgebauten schachtartigen Mittelteil sowie einem Deckelteil mit einer Einfüllöffnung gebildeten Aufnahmebehälter für Ausgangsmaterialien, dadurch gekennzeichnet, daß der Bodenteil (1) mindestens eine durch eine gelochte Platte (9) fest übergriffene Aussparung (3,5) und eine durch eine verschiebliche ungelochte Platte (10) übergriffene Aussparung (4) aufweist, daß in den Aussparungen (3,4,5) Schubladen (6 und 7) verschieblich eingestellt sind, daß die Schublade (6) der Aussparung (3,5) im Abstand hintereinander eine äußere, sich über die volle Höhe der Aussparung (3,5) erstreckende Querwand (32) und eine innere über eine Teilhöhe sich erstreckende Querwand (33) aufweist und daß der Boden (34) der Schublade (6,7) zwischen den Querwänden (32,33) offen ausgebildet ist.

2. Vorrichtung nach Anspruch 1, dadurch gekennzeichnet, daß der Bodenteil (1) eine durch eine ungelochte Platte (10) übergriffene Aussparung (4) und beidseitig zu dieser im Abstand je eine durch gelochte Platten (9) übergriffene Aussparung (3,5) aufweist.

3. Vorrichtung nach Anspruch 1 und 2, dadurch gekennzeichnet, daß der Bodenteil (1) an der Unterseite als Stützfüße dienende Ansätze (2), Anformungen aufweist.

4. Vorrichtung nach Anspruch 1, dadurch gekennzeichnet, daß die die Aussparungen (3 bis 5) übergreifenden Platten (6, 7) in in den Randbereichen der Aussparungen (3 bis 5) angeordnete Absetzungen (8) gelagert sind.

5. Vorrichtung nach Anspruch 1, dadurch gekennzeichnet, daß die Aussparung (4) bzw. die in dieser eingestellte Schublade (7) der Aufnahme von Regenwürmern dienen und mit dem Inneraum des Aufnahmebehälters (16) durch Verschieben der ungelochten Platte (10) in der Plattenebene verbindbar sind.

6. Vorrichtung nach Anspruch 1, dadurch gekennzeichnet, daß in den Aussparungen (3, 5) oder den in diesen untergebrachten Schubladen (6) Wärmeelemente austauschbar untergebracht sind.

7. Vorrichtung nach Anspruch 1, dadurch gekennzeichnet, daß der Bodenteil (1), die Elemente (11) und der Deckelteil (12) aus einem Isolierwerkstoff gebildet sind.

8. Vorrichtung nach Anspruch 7, dadurch gekennzeichnet, daß als Isolierwerkstoff ein aufgeschäumter Kunststoff oder ein Fasernwerkstoff dient.

9. Vorrichtung nach Anspruch 1, dadurch gekennzeichnet, daß der Bodenteil (1), die Elemente (11) und der Deckelteil (12) aus einem Werkstoff großer Festigkeit, z.B. Metall gebildet sind.

10. Vorrichtung nach Anspruch 1, dadurch gekennzeichnet, daß der Bodenteil (1), die Elemente (11) und der Deckelteil (12) in den jeweils gemeinsamen Trennungsflächen korrespondierende Nuten (13) und Federn (14) aufweisen und daß die Nuten (13) und Federn (14) den Bodenteil (1), die Elemente (11) und den Deckelteil (12) in der aufrechten Längsachse des Aufnahmebehälters (16) fixieren.

11. Vorrichtung nach Anspruch 10, dadurch gekennzeichnet, daß die in den Trennungsflächen angeordneten Nuten (13) und Federn (14) umlaufend ausgebildet sind.

12. Vorrichtung nach Anspruch 10, dadurch gekennzeichnet, daß in den gemeinsamen Trennungsflächen von Bodenteil (1), Elemente (11) und Deckelteil (12) abschnittsweise Abstandsstücke (36) angeordnet sind und daß die Bereiche zwischen den Abstandsstücken Zuluftöffnungen (15) für Außenluft bilden.

13. Vorrichtung nach Anspruch 12, dadurch gekennzeichnet, daß die Abstandsstücke (36) in den Eckbereichen der gemeinsamen Trennungsflächen von Bodenteil (1), Elemente (11) und Deckelteil (12) angeordnet sind.

14. Vorrichtung nach Anspruch 10, dadurch gekennzeichnet, daß der Bodenteil (1) und/oder die Elemente (11) innen nach oben die obere Begrenzungsflächen derselben überragende gelochte Streifen (17) tragen, die mittels in Aussparungen (18') der Begrenzungsflächen einragende Anformungen (18) am Bodenteil und/oder den Elementen festgelegt sind.

15. Vorrichtung nach Anspruch 1, dadurch gekennzeichnet, daß der Bodenteil (1) und/oder die Elemente (11) in den Umfangsflächen Durchführungsöffnungen (22) für Temperaturmeßeinrichtungen (23) aufweisen.

16. Vorrichtung nach Anspruch 1, dadurch gekennzeichnet, daß der Deckelteil (12) eine durch einen Klapp- oder Stülpdeckel verschließbare Öffnung aufweist.

17. Vorrichtung nach Anspruch 1, dadurch gekennzeichnet, daß der Deckelteil (12) in einer Öffnung (24) einen mit einer Schneid- oder Schlageinrichtung (27) versehenen Häcksler (25) für die Ausgangsstoffe aufweist.

18. Vorrichtung nach Anspruch 1, dadurch gekennzeichnet, daß im Bodenteil (1) zwischen den Stützfüßen (2) Einhängeschlitze (20) für Tragegriffe (21) ausgebildet sind.

19. Vorrichtung nach Anspruch 1, dadurch gekennzeichnet, daß die Elemente (11) Stecköffnungen (29) für die Durchführung von mit einer Wärmepumpe verbindbaren Wärmezellen (30, 31) aufweisen (Fig. 2).

20. Vorrichtung nach Anspruch 1, dadurch gekennzeichnet, daß der Bodenteil (1) und/oder Deckelteil (12) und/oder die Elemente (11) innen mit einem Folienwerkstoff kaschiert sind.

21. Vorrichtung nach Anspruch 1, dadurch gekennzeichnet, daß der Bodenteil (1), die Elemente (11) und der Deckelteil (12) eine eckige oder runde Querschnittsform aufweisen.

22. Vorrichtung nach Anspruch 1, dadurch gekennzeichnet, daß der Bodenteil (1) die Elemente (11) und der Deckelteil (12) außen eine Wetterschutzauflage aufweisen.

23. Vorrichtung nach Anspruch 1, dadurch gekennzeichnet, daß die Elemente (11) in der inneren Begrenzungsfläche zum Innenraum des Aufnahmebehälters (16) offene und in Längsrichtung desselben sich erstreckende Luftführungskanäle (38) aufweisen.

**Claims**

1. Arrangement for producing humus, including a receptacle for starting materials, which receptacle is formed by a lower portion having an air inlet aperture, a central compartment-like portion comprising elements, and an upper portion having a feed aperture, characterised in that the lower portion (1) has at least one recess (3, 5), which is securely covered by a perforated plate (9), and a recess (4), which is covered by a displaceable, non-perforated plate (10), in that drawers (6 and 7) are displaceably inserted in the recesses (3, 4, 5), in that the drawer (6) of the recess (3, 5) has an outer transverse wall (32), which extends to the full height of the recess (3, 5), and an inner transverse wall (33), which extends over part of the height, such walls being disposed one behind the other with a spacing therebetween, and in that the base (34) of the drawer (6, 7) is open between the transverse walls (32, 33).

2. Arrangement according to claim 1, characterised in that the lower portion (1) has a recess (4), which is covered by a non-perforated plate (10), and a recess (3, 5) on each side of said recess (4) with a spacing therebetween, such recesses being covered by perforated plates (9).

3. Arrangement according to claims 1 and 2, characterised in that the lower portion (1) is provided, on its underside, with projection members (2) - moulded-on portions - which serve as supporting feet.

4. Arrangement according to claim 1, characterised in that the plates (9, 10), which cover the recesses (3 to 5), are mounted in shoulder portions (8), which are disposed in the edge regions of the recesses (3 to 5).

5. Arrangement according to claim 1, characterised in that the recess (4), or respectively the drawer (7) inserted therein, serves to accommodate earthworms and is connectable to the interior of the receptacle (16) by displacement of the non-perforated plate (10) in the plate plane.

6. Arrangement according to claim 1, characterised in that heating elements are interchangeably accommodated in the recesses (3, 5) or in the drawers (6) accommodated therein.

7. Arrangement according to claim 1, characterised in that the lower portion (1), the elements (11) and the upper portion (12) are formed from an insulating material.

8. Arrangement according to claim 7, characterised in that an expanded plastics material or a fibrous material serves as the insulating material.

9. Arrangement according to claim 1, characterised in that the lower portion (1), the elements (11) and the upper portion (12) are formed from a high-tensile material, e.g. metal.

10. Arrangement according to claim 1, characterised in that the lower portion (1), the elements (11) and the upper portion (12) have corresponding grooves (13) and tongues (14) in their respective common separation faces, and in that the grooves (13) and tongues (14) secure the lower portion (1), the elements (11) and the upper portion (12) in the upwardly extending longitudinal axis of the receptacle (16).

11. Arrangement according to claim 10, characterised in that the grooves (13) and tongues (14), which are disposed in the separation faces, are circumferential.

12. Arrangement according to claim 10, characterised in that spacer members (36) are disposed at intervals in the common separation faces of lower portion (1), elements (11) and upper portion (12), and in that the regions between the spacer members form air inlet apertures (15) for outside air.

13. Arrangement according to claim 12, characterised in that the spacer members (36) are disposed in the corner regions of the common separation faces of lower portion (1), elements (11) and upper portion (12).

14. Arrangement according to claim 10, characterised in that the lower portion (1) and/or the elements (11) are provided with perforated strips (17), which protrude internally upwardly beyond the upper boundary faces thereof and are secured on the lower portion and/or the elements by means of moulded-on portions (18), which extend into recesses (18') in the boundary faces.

15. Arrangement according to claim 1, characterised in that the lower portion (1) and/or the elements (11) are provided in the circumferential faces with through-apertures (22) for temperature measuring means (23).

16. Arrangement according to claim 1, characterised in that the upper portion (12) has an aperture which is closable by means of a hinged or pivotable cover.

17. Arrangement according to claim 1, characterised in that the upper portion (12) has, in an aperture (24), a chopper (25) for chopping-up the starting materials, which chopper is provided with a cutting or compacting means (27).

18. Arrangement according to claim 1, characterised in that mounting slots (20) for the insertion of carrying handles (21) are provided in the lower portion (1) between the supporting feet (2).

19. Arrangement according to claim 1, characterised in that the elements (11) have insert apertures (29) through which heater cells (30, 31) pass, such cells being connectable to a heater pump (Fig. 2).

20. Arrangement according to claim 1, characterised in that the lower portion (1) and/or upper portion (12) and/or the elements (11) are internally lined with a film-like material.

21. Arrangement according to claim 1, characterised in that the lower portion (1), the elements (11) and the upper portion (12) have an angular or circular cross-sectional configuration.

22. Arrangement according to claim 1, characterised in that the lower portion (1), the elements (11) and the upper portion (12) are externally provided with a weatherproof covering.

23. Arrangement according to claim 1, characterised in that the elements (11) have, in the inner boundary face, airflow channels (38), which are open towards the interior of the receptacle (16) and extend in the longitudinal direction thereof.

## Revendications

1. Dispositif pour fabriquer de l'humus, comportant un réceptacle pour des matières de départ, formé par une partie de fond à ouverture d'air amené, une partie centrale en forme de cheminée fabriquée à partir d'éléments, ainsi qu une partie de couverture présentant une ouverture de remplissage,
caractérisé en ce que la partie de fond (1) présente au moins un évidement (3,5) recouvert, de façon fixe, par une plaque perforée (9) et un évidement (4) recouvert par une plaque (10) non perforée mobile, en ce que, dans les évidements (3,4,5), sont montés, de façon mobile, des tiroirs (6 et 7), en ce que le tiroir (6) de l'évidement (3,5) présente, à distance l'une derrière l'autre, une paroi transversale externe (32) s'étendant sur toute la hauteur de l'évidement (3,5) et une paroi transversale interne (33) s étendant sur une hauteur partielle, et en ce que le fond (34) du tiroir (6,7) est ouvert entre les parois transversales (32,33).

2. Dispositif selon la revendication 1,
caractérisé en ce que la partie de fond (1) présente un évidement (4) recouvert par une plaque non perforée (10) et, des deux côtés par rapport à celui-ci à distance, à chaque fois un évidement (3,5) recouvert par une plaque perforée (9).

3. Dispositif selon les revendications 1 et 2, caractérisé en ce que la partie de fond (1) présente, sur son dessous, des parties conformées, réalisées en tant que pièces rapportées (2) servant de pieds d'appui.

4. Dispositif selon la revendication 1,

caractérisé en ce que les plaques (9,10) recouvrant les évidements (3 à 5) sont montées dans des retraits (8) agencés dans les zones marginales des évidements (3 à 5).

5. Dispositif selon la revendication 1, caractérisé en ce que l'évidement (4) et le tiroir (7) monté dans celui-ci servent à recevoir des vers de terre et peuvent être reliés avec l'espace interne du réceptacle (16) par déplacement de la plaque non perforée (10) dans le plan de la plaque.

6. Dispositif selon la revendication 1, caractérisé en ce que, dans les évidements (3,5) ou dans les tiroirs (6) agencés dans ceux-ci, sont disposés, de façon interchangeable, des éléments thermiques.

7. Dispositif selon la revendication 1, caractérisé en ce que la partie de fond (1), les éléments (11), et la partie de couverture (12) sont réalisés en un matériau isolant.

8. Dispositif selon la revendication 7, caractérisé en ce que, en tant que matériau isolant, est utilisée une matière synthétique expansée ou une matière à base de fibres.

9. Dispositif selon la revendication 1, caractérisé en ce que la partie de fond (1), les éléments (11), et la partie de couverture (12) sont formés en une matière de résistance mécanique élevée, par exemple un métal.

10. Dispositif selon la revendication 1, caractérisé en ce que la partie de fond (1), les éléments (11), et la partie de couverture (12) présentent des rainures (13) et des languettes (14) correspondantes dans leurs surfaces de séparation communes, et en ce que les rainures (13) et les languettes (14) fixent la partie de fond (1), les éléments (11), et la partie de couverture (12) par rapport à l'axe longitudinal vertical du réceptacle (16).

11. Dispositif selon la revendication 10, caractérisé en ce que les rainures (13) et les languettes (14) agencées dans les surfaces de séparation sont réalisés sur toute la périphérie.

12. Dispositif selon la revendication 10, caractérisé en ce que, dans les surfaces de séparation communes de la partie de fond (1), des éléments (11) et de la partie de couverture (12), sont agencées, de façon tronçonnée, des pièces d'écartement (36), et en ce que les zones entre les pièces d'écartement forment des ouvertures (15) pour l'amenée de l'air extérieur.

13. Dispositif selon la revendication 12, caractérisé en ce que les pièces d'écartement (36) sont agencées dans les zones de coin des surfaces de séparation communes de la partie de fond (1), des éléments (11), et de la partie de couverture (12).

14. Dispositif selon la revendication 10, caractérisé en ce que la partie de fond (1) et/ou les éléments (11) portent, intérieurement, des bandes (17) perforées faisant saillie vers le haut des surfaces de limitation supérieures de ceux-ci, bandes qui sont fixées à la partie de fond et/ou aux éléments par des pièces rapportées (18) s'engageant dans des évidements (18') des surfaces de limitation.

15. Dispositif selon la revendication 1, caractérisé en ce que la partie de fond (1) et/ou les éléments (11) présentent, dans leurs surfaces périphériques, des ouvertures de passage (22) pour des dispositifs de mesure de la température (23).

16. Dispositif selon la revendication 1, caractérisé en ce que la partie de couverture (12) présente une ouverture qui peut être fermée par un couvercle rabattable ou à recouvrement.

17. Dispositif selon la revendication 1, caractérisé en ce que la partie de couverture (12) présente, dans une ouverture (24), un hachoir (25) muni d'un dispositif de coupe ou de percussion (27) pour les matières de départ.

18. Dispositif selon la revendication 1, caractérisé en ce que, dans la partie de fond (1) entre les pieds d'appui (2), sont réalisées des fentes de suspension (20) pour des moyens de support (21).

19. Dispositif selon la revendication 1, caractérisé en ce que les éléments (11) présentent des ouvertures (29) pour le passage de cellules thermiques (30,31) qui peuvent être reliées à une pompe à chaleur (figure 2).

20. Dispositif selon la revendication 1, caractérisé en ce que la partie de fond (1) et/ou la partie de couverture (12) et/ou les éléments (11) sont doublés, intérieurement, d'un matériau en feuille.

**21.** Dispositif selon la revendication 1, caractérisé en ce que la partie de fond (1), les éléments (11), et la partie de couverture (12) présentent une forme de section transversale ronde ou polygonale.

**22.** Dispositif selon la revendication 1, caractérisé en ce que la partie de fond (1), les éléments (11), et la partie de couverture (12) présentent, extérieurement, un revêtement de protection contre les intempéries.

**23.** Dispositif selon la revendication 1, caractérisé en ce que les éléments (11), dans la surface de limitation interne, présentent des canaux (38) de guidage d'air ouverts vers l'espace interne du réceptacle (16) et s'étendant en direction longitudinale de celui-ci.

Fig. 1

Fig. 4

Fig. 2

Fig. 3

Fig.5

Fig.6

Fig.7

Fig.8

Fig.9

Fig.11

Fig.10

Fig.12

Fig.13

11

Fig. 14

Fig. 15

Fig. 16

Fig. 17